# EUROPEAN PATENT SPECIFICATION

(11) **EP 2 445 453 B1**
(45) Date of publication and mention of the grant of the patent: **11.12.2013**
(21) Application number: 10747305.0
(22) Date of filing: 23.06.2010
(51) Int. Cl.: A61F 2/46

(54) **DEVICE FOR POSITIONING A BONE MATRIX OR A BONE SCAFFOLD**
VORRICHTUNG ZUR POSITIONIERUNG EINER KNOCHENMATRIX ODER EINES KNOCHENGERÜSTES
DISPOSITIF DE POSITIONNEMENT DE MATRICE OSSEUSE OU D'ÉCHAFAUDAGE OSSEUX

(30) Priority: 23.06.2009 IT RM20090323
(43) Date of publication of application: 02.05.2012
(73) Proprietor: Calori, Giorgio Maria, 20123 Milano (IT); Brenicci, Tommaso, 20090 Segrate (MI) (IT)
(72) Inventor: Calori, Giorgio Maria, 20123 Milano (IT); Brenicci, Tommaso, 20090 Segrate (MI) (IT)
(74) Representative: Tiburzi, Andrea
(86) International application number: PCT/IT2010/000278
(87) International publication number: WO 2010/150294

(56) References cited:
- EP-A- 1 820 477
- US-A1- 2003 236 573
- US-B1- 7 530 982

## Description

The present invention relates to a device for positioning a bone matrix or a bone scaffold in avascular necrosis.

More specifically, the invention concerns a device for insertion of a bone matrix or of a bone scaffold for treating avascular necrosis of femur head and of other physical parts.

As it is well known, at present, femur head avascular necrosis (please note that, although specification is addressed to the femur head avascular necrosis, the following considerations are also valid for other physical parts) is a relatively common hip disease, during which it is observed death of cells populating a variously defined area of femur head. Femur head (hip) is usually subjected to this kind of disease. Femur at the knee level and humeral head (shoulder) are less frequently interested by this disease, while astragal body, carpale scaphoid and navicular bone are more rarely involved.

It must be taken into consideration that 5% of hip osteoarthrosys requiring prosthesis is caused by avascular necrosis. Usually, peak of the disease is between patients 30 years old and patients 60 years old.

Many possible causes have been indicated in literature for femur head avascular necrosis: infective, traumatic, chronic use of corticosteroids for arthritis deformans, lupus erytematous systemic or atopic dermatitis, obesity, toxic substances, alcohol abuse and post-pregnancy. Moreover, sometimes, these etiopathogenetic agents are found associated each other. Last, but not least, it is mentioned osteonecrosis on idiopathic base.

Extension of avascular necrosis is a function of the tissue circulation compromising degree.

During the first stage of the disease, nuclear magnetic resonance (NMR) is the main and most efficient diagnosis method. Radiographic signs appear later on with respect to the pain, sometimes even after six months. Diagnosis is mainly based on clinical signs, since traditional radiography and scintigraphy can both be negative at the first femur head avascular necrosis stages.

Moreover, magnetic resonance can provide a reliable estimate of femur h ead lesion extension, due since the first transformations of transformations of hemopoietic brown cells into fat cells, thus evidencing those patients with high osteonecrosis risk.

As a consequence of cellular death, bone area interested is no more subjected to renewal phenomenon ensured by cells and are subjected to structural alteration with destruction of trabecular structure, collapse and reabsorption of bone tissue.
Furthermore, on the basis of dimensions of interested area and of its position, more or less serious consequences can exist about integrity and function of hip articulation. At present, pathology under examination is treated by hip arthroprosthesis. The patent US 7530982 B1 describes a device for positioning a bone matrix or a bone scaffold according to the preamble of claim 1.

In view of the above, it is object of the present invention that of suggesting a device permitting a treatment which is an alternative to the described pathology, in order to permit regeneration of bone tissue by an equine bone scaffold obtained by an exclusive process of enzymatic de-anti-generation, permitting fully eliminating antigenic component of tissue without modifying mineral component and bone collagen, on which it is deposited. Substances are joined to the matrix for stimulating tissue regeneration, such as recombinant morphogenic protein, growth factors, and stremal cells.

The present invention is a device for positioning a bone matrix or a bone scaffold in an anatomic seat to be subjected to treatment, comprising a cannula wherein said bone matrix or bone scaffold can be inserted beforehand, said cannula having a first end, suitable to reach said anatomic seat, and a second end, and a piston, inserted in said cannula whereby said bone matrix or bone scaffold are ejected through said first end from said cannula wherein said piston has a channel along its longitudinal axis for the insertion and the passage of liquid substances

In a preferred embodiment of the invention, said second end is provided with a first handle and a second handle is provided at an end of said piston.

In a preferred embodiment of the invention, said device comprises a receptacle, fixed with said cannula, for preparing a liquid mixture that comprises growth factors and/or mesenchymal cells and/or staminal cells.

Furthermore, in a preferred embodiment of the invention, said receptacle is fixed close to said second end of said cannula.

In a preferred embodiment of the invention, said piston is provided with reference indentations for indicating the depth of insertion of said piston in said cannula.

The present invention will be described in the following for illustrative but not limitative purposes, according to its preferred embodiments, with particular reference to the enclosed figures of the drawings, wherein:
figure 1 is a perspective view of device for positioning a bone matrix or a bone scaffold in avascular necrosis, according to the invention;
figure 2shows a lateral view of a portion of device of figure 1; and
   figure 3 is a top view of device of figure 1.

The same reference numbers will be used in the different figures to indicate similar parts.

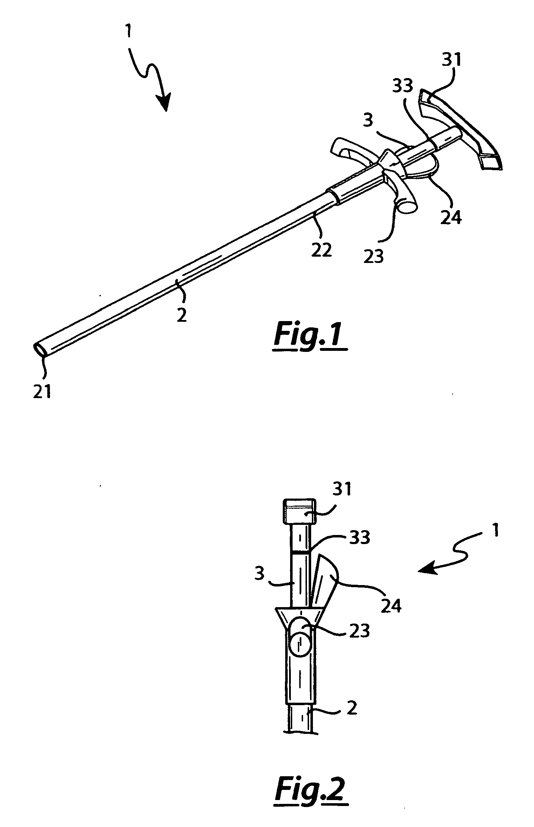

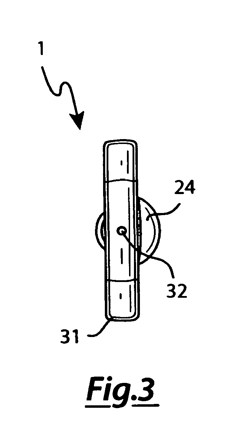

Making reference to figures 1 - 3, it is observed a device 1 for positioning a bone matrix or a bone scaffold according to the present invention, to be used for avascular necrosis, comprising a cannula 2, having a first end21 and a second end 22. Said first end 21 can be inserted within the anatomical seat to be subjected to treatment, i.e. wherein said bone matrix or said bone scaffold must be introduced. Said cannula 2 also comprises a first handle 23 fixed to said second end 22.

Bone matrix or bone scaffold can be inserted within cannula 2 for tissue treatment.

Device 1 also comprises a piston 3 inserted within said cannula 2. Said piston 3 also comprises a second handle 31. Said bone matrix or said bone scaffold can be ejected by said piston 3 from said cannula 2, and particularly from said first end 21.

Said piston 3 also comprises a channel 32 longitudinally passing, through which operator can introduce liquid substances within device 1.

Piston 3 is finally provided with reference notches 33, so as to indicate introduction depth of said piston 3 within said cannula 2.

Said cannula 2 also comprised a container 24 aimed to prepare a liquid mixture comprising growth factors and/or mesenchymal cells and/or staminal cells. Said mixture can be used for enriching said bone matrix or said bone scaffold, on the basis of the clinical indication.

When necrotic tissue is removed and extracted by a mill or a suitable surgical instrument, cannula 2 is inserted by said handle 23.

Said bone matrix or bone scaffold is preloaded within device 1 by cutaneous accession.

Under radiographic control, said cannula 2 first end 21 is positioned within the anatomic seat to be subjected to treatment.

Thanks to notches 33 on piston 3, it is possible checking insertion depth and individuating position in which bone matrix of bone scaffold is in touch with the anatomic seat to be subjected to treatment.

By a further pressure of piston 3, it is possible releasing said bone matrix or bone scaffold outside cannula 2, withdrawing device 1, and thus easing diffusion of recombinant morphogenetic proteins all around necrotic area.

Bone matrix or bone scaffold, rehydrated and enriched with growth factors or mesenchymal cells, can be compressed to conform to geometry within cannula 2. Such an arrangement permits to flexible matrix, once brought to the end of cannula 2, adhering to the bone seat already prepared beforehand, and preventing scaffold migration phenomenon.

It is further possible inserting, by device 1, more than one bone matrixes or scaffolds in order to repair tissue damages of spongy tissue.

Present invention has been described for illustrative, but not limitative purposes, according to its preferred embodiments, but it is understood that variations and/or modifications can be introduced by those skilled in the art without departing from the relevant scope, as defined in the enclosed claims.

## Claims

1. Device (1) for positioning a bone matrix or a bone scaffold in an anatomic seat to be subjected to treatment, comprising
a cannula (2) wherein said bone matrix or bone scaffold can be inserted beforehand, said cannula (2) having a first end (21), suitable to reach said anatomic seat, and a second end (22), and
a piston (3), inserted in said cannula (2) whereby said bone matrix or bone scaffold are ejected through said first end (21) from said cannula (2),
**characterized in that** said piston (3) has a channel along its longitudinal axis for the insertion and the passage of liquid substances.

2. Device (1) according to claim 1, **characterized in that** said second end (22) is provided with a first handle (23) and a second handle is provided at an end (31) of said piston (3).

3. Device (1) according to anyone of the preceding claims, **characterized in that** it comprises a receptacle (24), fixed with said cannula (2).

4. Device (1) according to anyone of the preceding claims, **characterized in that** said receptacle (24) is fixed close to said second end (22) of said cannula (2).

5. Device (1) according to anyone of the preceding claims, **characterized in that** said piston (3) is provided with reference indentations (33) for indicating the depth of insertion of said piston (3) in said cannula (2).

## Patentansprüche

1. Vorrichtung (1) zur Positionierung einer Knochenmatrix oder eines Knochengerüsts an einen anatomischen Platz, der einer Behandlung unterzogen werden soll, umfassend:
eine Kanüle (2), worin die Knochenmatrix oder das Knochengerüst im Voraus eingeführt werden kann, wobei die Kanüle (2) ein erstes Ende (21), das geeignet ist, den anatomischen Platz zu erreichen, und ein zweites Ende (22) aufweist, und
einen Kolben (3), der in die Kanüle (2) eingeführt ist, wobei die Knochenmatrix oder das Knochengerüst durch das erste Ende (21) von der Kanüle (2) ausgestoßen werden,
**dadurch gekennzeichnet, dass** der Kolben (3) entlang seiner longitudinalen Achse einen Kanal für die Einführung und den Durchtritt von flüssigen Substanzen aufweist.

2. Vorrichtung (1) gemäß Anspruch 1, **dadurch gekennzeichnet, dass** das zweite Ende (22) mit einem ersten Griff (23) versehen ist und ein zweiter Griff an einem Ende (31) des Kolbens (3) vorgesehen ist.

3. Vorrichtung (1) gemäß einem der vorhergehenden Ansprüche, **dadurch gekennzeichnet, dass** sie eine Aufnahme (24) umfasst, die bei der Kanüle (2) befestigt ist.

4. Vorrichtung (1) gemäß einem der vorhergehenden Ansprüche, **dadurch gekennzeichnet, dass** die Aufnahme (24) nahe dem zweiten Ende (22) der Kanüle (2) befestigt ist.

5. Vorrichtung (1) gemäß einem der vorhergehenden Ansprüche, **dadurch gekennzeichnet, dass** der Kolben (3) mit Bezugsmarkierungen (33) zum Anzeigen der Tiefe der Einführung des Kolbens (3) in die Kanüle (2) versehen ist.

## Revendications

1. Dispositif (1) pour positionner une matrice osseuse ou une charpente osseuse dans un siège anatomique qui doit être soumis à un traitement, comprenant
une canule (2) dans laquelle ladite matrice osseuse ou charpente osseuse peut être préalablement insérée, ladite canule (2) ayant une première extrémité (21) appropriée pour atteindre ledit siège anatomique, et une seconde extrémité (22), et
un piston (3), inséré dans ladite canule (2), de sorte que ladite matrice osseuse ou charpente osseuse est éjectée de ladite canule (2) à travers ladite première extrémité (21),
**caractérisé en ce que** ledit piston (3) présente le long de son axe longitudinal un canal prévu pour l'introduction et le passage de substances liquides.

2. Dispositif (1) selon la revendication 1, **caractérisé en ce que** ladite seconde extrémité (22) est munie d'une première poignée (23) et **en ce qu'**une seconde poignée est prévue à une extrémité (31) dudit piston (3).

3. Dispositif selon une quelconque des revendications précédentes, **caractérisé en ce qu'**il comprend un récipient (24) fixé à ladite canule (2).

4. Dispositif selon une quelconque des revendications précédentes, **caractérisé en ce que** ledit récipient (24) est fixé à proximité de ladite seconde extrémité (22) de ladite canule (2).

5. Dispositif selon une quelconque des revendications précédentes, **caractérisé en ce que** ledit piston (3) est muni d'encoches de référence (33) prévues pour indiquer la profondeur d'insertion dudit piston (3) dans ladite canule (2).
